# EUROPEAN PATENT SPECIFICATION

(11) **EP 4 277 673 B1**
(45) Date of publication and mention of the grant of the patent: **31.12.2025**
(21) Application number: 22739275.0
(22) Date of filing: 18.01.2022
(51) Int. Cl.: A61L 27/20, A61L 27/22, A61L 27/26, A61L 27/36, A61L 27/38, A61L 27/50, B29C 67/02, B33Y 70/00, B33Y 80/00, C09D 11/04, C09D 11/14

(54) **A SODIUM ALGINATE, GELATIN, COLLAGEN AND FIBRIN (AGCF) BASED BIO-INK FOR THE BIOPRINTING OF A 3D BIOGEL-BASED TISSUE/STRUCTURE**
AUF NATRIUMALGINAT, GELATINE, KOLLAGEN UND FIBRIN (AGDF) BASIERENDE BIOTINTE ZUM BIODRUCKEN EINES 3D-BIOGELBASIERTEN GEWEBES/EINER BIOGELBASIERTEN STRUKTUR
BIO-ENCRE À BASE D'ALGINATE DE SODIUM, DE GÉLATINE, DE COLLAGÈNE ET DE FIBRINE (AGCF) POUR LA BIO-IMPRESSION D'UN TISSU/D'UNE STRUCTURE À BASE DE BIOGEL 3D

(30) Priority: 18.01.2021 US 202163138534 P
(43) Date of publication of application: 22.11.2023
(73) Proprietor: The State of Israel, Ministry of Agriculture & Rural Development, Agricultural Research Organization (ARO) (Volcani Institute), 7528809 Rishon-Lezion (IL)
(72) Inventor: KOLTAI, Hinanit, 75284 Rishon LeZion (IL); SHALEV, Nurit, Tirat Yehuda (IL); AJJAMPURA, Vinayaka, Chandrashekarappa, 577547 Karnataka (IN)
(74) Representative: Jaeger, Michael David
(86) International application number: PCT/IL2022/050070
(87) International publication number: WO 2022/153319

(56) References cited:
- EP-A1- 3 326 661
- WO-A1-2019/245110
- US-A1- 2012 089 238
- WLODARCZYK-BIEGUN MALGORZATA K ET AL: "3D bioprinting of structural proteins", BIOMATERIALS, ELSEVIER, AMSTERDAM, NL, vol. 134, 12 April 2017 (2017-04-12), pages 180 - 201, XP085004461, ISSN: 0142-9612, DOI: 10.1016/J.BIOMATERIALS.2017.04.019

## Description

### FIELD OF THE INVENTION

The invention is in the field of bioprinting and tissue engineering

### BACKROUND OF THE INVENTION

Bioprinting has been developed as a new method of tissue engendering, specifically for the manufacturing of 3-dimentional structures. In some cases, the structures are used as a base for the biological material, such as cell. Recent methods for the 3-D printing, also known as additive manufacturing, have problems in accuracy and stability.

US 2012/089238 discloses a cell carrier material comprising four separate components: gelatin, fibrinogen, glycerol, and hyaluronic acid. Several ratios of these components have been tested, and the combination of 20 mg/ml gelatin, 3 mg/ml hyaluronic acid, 10 mg/ml fibrinogen, and 10% v/v glycerol was selected for further study. However, US 2012/089238 does not suggest any additional reaction steps to improve the stability of the bio-ink. Instead, the preparation of the cell carrier material was based solely on the simple mixing of components without any prior reaction.

There is a long felt need for a composition and method for the manufacturing of tissue.

### SUMMARY

It is an object of the present invention to present a bio-ink comprising:
a. a mixture of gelatin and sodium alginate;
b. at least one structural protein selected from a group consisting of collagen, vitronectin, laminins and fibronectin; and
c. fibrinogen;
wherein the mixture is pre-heated to a temperature in the range of 50-80°C.

It is another object of the present invention to present the bio-ink as presented in any of the above, wherein the at least structural protein is collagen, and the ratio of the gelatin to the collagen is approximately 15:1 to 25:1

It is another object of the present invention to present the bio-ink as presented in any of the above, wherein the ratio of the fibrinogen to the collagen is at a ratio of 3:1 to 1:3.

It is another object of the present invention to present the bio-ink as presented in any of the above, additionally comprising cells.

It is another object of the present invention to present the bio-ink as presented in any of the above, wherein said cells are characterized by being viable/alive.

It is another object of the present invention to present the bio-ink as presented in any of the above, additionally comprising at least one crosslinking agent, wherein at least one of the following is true:
a. the crosslinking agent is selected from a group consisting of thrombin, calcium, copper, magnesium, manganese, strontium, barium, aluminum or a combination thereof; and
b. the crosslinking agent is a serine protease.

It is another object of the present invention to present the bio-ink as presented in any of the above, additionally comprising salts, acids, bases and buffer solutions, further wherein one of the following is true:
a. the salt is a calcium salt, a copper salt, magnesium salt, manganese salt, a strontium salt, a barium salt, an aluminum salt and a combination thereof; and
b. the salt is a chloride salt or a sulfate.

It is another object of the present invention to present a bio-ink as presented in any of the above, wherein the copper salt selected from a group consisting of copper (II) sulfate (CuSO4), copper bromide (CuBr₂), copper fluoride (CuF₂), copper carbonate (CuCO₃), Copper nitrate (CuNO₃)₂, Copper oxide (CuO), Copper acetate Cu(OAc)₂ and Copper azide Cu(N₃)₂.

It is another object of the present invention to present the bio-ink as presented in any of the above, characterized by at least one of the following:
a. viscosity in a range of 1-20000 Pa. S;
b. cell proliferation of ≥80%;
c. cell viability of ≥80%;
d. soluble in 0.05M EDTA+0.05M sodium citrate;
e. pH of 6.5-7.4; and
f. a boiling point of >100°C;

It is another object of the present invention to present a method of producing a bio-ink, comprising steps of:
a. preparing a solution of sodium alginate and gelatin;
b. preparing a solution of fibrinogen;
c. preparing a solution of collagen;
d. mixing the fibrinogen and collagen solutions;
e. adding cells to the fibrinogen and collagen solution; and,
f. mixing the cells, fibrinogen and collagen solution with the Sodium alginate and gelatin solution;
wherein step (a) comprises heating the solution of the sodium alginate and gelatin to a temperature in the range of 50-80°C.

It is another object of the present invention to present the method as presented in any of the above, additionally comprising at least one of the steps:
a. sterilizing said solutions;
b. filtering said solutions;
c. centrifuging said solutions; and
d. filtering said solutions.

It is another object of the present invention to present a 3D structure, comprising:
a. a mixture of gelatin and sodium alginate;
b. at least one structural protein selected from a group consisting of collagen, vitronectin, laminins and fibronectin;
c. viable cells;
d. fibrinogen; and
e. at least one crosslinking agent,
wherein the structure supports cell development and formation of secondary structures, further wherein the mixture is pre-heated to a temperature in the range of 50-80°C.

It is another object of the present invention to present the 3D structure as presented in any of the above, characterized by at least one of the following:
a. viscosity in a range of 1-20000 Pa. S;
b. cell viability of ≥80%;
c. soluble in 0.05M EDTA+0.05M sodium citrate;
d. stability at a temperature range of 35 to 42 °C;

It is another object of the present invention to present a method of printing a structure, comprising steps of:
a. obtaining the bio-ink as presented in any of the above;
b. obtaining a printer and a temperature-controlled printer head;
c. preparing the printer head;
d. printing the bio-ink;
e. adding at least one crosslinking agent; and
f. incubating the printed structure;

wherein the crosslinking agent coagulates the bio-ink;
further wherein the printing is characterized by at least one of the following:
   a. a temperature in a range of 20-25°C;
   b. a nozzle of 22G, 25G or 27G;
   c. a speed of 1 to 3 mm/sec;
   d. pressure of 10 to 20 kPa;
   e. an Infill density of <25%.

### BRIEF DESCRIPTION OF THE DRAWINGS

The accompanying drawings, which are included to provide a further understanding of the invention and are incorporated in and constitute a part of this specification, illustrate embodiments of the invention and together with the description serve to explain the principles of the invention wherein:
Figure 1 -presents (a) 24 well plate with rings printed as described above and contain GBM cells, (B) printed ring in 4X magnification.
Figure 2 - presents a confocal image of GBM cells (DAPI staining) 6 days after printing in AGCF. 200µm magnification.
Figure 3 - presents a confocal image of GBM cells (DAPI staining) 13 days after printing in AGCF. 200µm magnification.
Figure 4 - presents a confocal image of GBM cells (DAPI staining) 13 days after printing in AGCF: (A) 50 µm (B) 200 µm, (C) GBM sphere showed on day 13 at 10X magnification.

### DETAILED DESCRIPTION OF THE PERFERD EMBODYMENT

The following description is provided, alongside all chapters of the present invention, so as to enable any person skilled in the art to make use of the invention and sets forth the best modes contemplated by the inventor of carrying out this invention. Various modifications, however, are adapted to remain apparent to those skilled in the art, since the generic principles of the present invention have been defined specifically to provide compositions and methods.

In this application the term 'ink' or 'bio-ink' refers to printable biomaterials used in three dimensional (3D) bioprinting processes. In some embodiments, cells and other biologics are deposited in a spatially controlled pattern to fabricate living tissues and organs.

In this application the term 'crosslinker' refers to a compound (or compounds) that separately (or in combination) bond one polymer chain (or one part of a chain) to another polymer chain (or another section of the same part of a chain).

In this application the terms 'crosslinking agent' or 'crosslinking factor' refers to a compound (or compounds) that separately (or in combination) create a bond between one polymer chain (or one part of a chain) to another polymer chain (or another section of the same part of a chain).

Unless otherwise stated, with reference to numerical quantities, the term "about" refers to a tolerance of ±25% of the stated nominal value.

Unless otherwise stated, all numerical ranges are inclusive of the stated limits of the range.

### Detailed Description of the invention

Bioprinting systems commonly have three major segments: hardware (printer), bio-ink and printing template.

Three main types of printer technologies are use in bioprinting: inkjet, laser-assisted, and extrusion printers. Inkjet printers are mainly used for fast printing and large-scale products. One type of inkjet printer, called drop-on-demand inkjet printer, prints materials in exact amounts, minimizing cost and waste. Laser-assisted printing are commonly used to provide high-resolution printing. Extrusion printers print cells layer-by-layer to create 3D constructs.

The bio-ink serves to create a 3-D structure, that serves as a basis for the growth delivery medium for (living) cells, that behaves much like a liquid, enabling the printing of the desired shape. The components of the ink of the present invention can be divided into 2 main groups according to the function:
- Structural components, that can be sub-divided by chemical classification:
   ∘ Structural polysaccharides:
      - Sodium alginate, also called alginic acid, is a polysaccharide distributed widely in the cell walls of brown algae that is hydrophilic and forms a viscous gum when hydrated. Sodium alginate is a linear copolymer with homopolymeric blocks of (1-4)-linked β-D-mannuronate (M) and its C-5 epimer α-L-guluronate (G) residues. The M and G blocks may appear as alternates or consecutive.
   ∘ Structural proteins (also known as Scleroproteins or fibrous proteins) are commonly constructed by elongated or fibrous polypeptide chains which form filamentous and sheet like structure. Scleroproteins typically have low solubility in water. In some embodiments, the structural proteins also promote cell attachment and proliferation.
      - Collagen is the main structural protein in the extracellular matrix in the various connective tissues in the body and consists of amino acids bound together to form a triple helix of elongated fibril (known as a collagen helix). Collagen is the most abundant protein in mammals, making up from 25% to 35% of the whole-body protein content. Collagen forms a triple helix of elongated fibril^{,} known as a collagen helix. The hardness or rigidity of the collagen tissue depending upon the degree of mineralization, ranging from rigid (such as bones) to compliant (such as a tendon) or as a gradient covering the range from rigid to compliant (such as cartilage). The Collagen could be of type I-V and from various (mammalian) sources, such as calf skin.
      - Gelatin (also referred to as gelatine, hydrolyzed collagen, collagen hydrolysate, gelatine hydrolysate and hydrolyzed gelatine) is a translucent, colorless and flavorless material often used as a food ingredient. Gelatin is derived from collagen that has been partially purified and hydrolyzed. Collagen hydrolysis is performed by one of three different methods: acid-, alkali-, and enzymatic hydrolysis. Gelatin is brittle when dry and gummy when moist. Gelatin forms thermally reversible gels with water and can form stable foams.
         Both collagen and gelatin are 98-99% protein by dry weight, with the amino acid content being24-25% proline and/or hydroxyproline, 20-21% glycine, 10-11% glutamic acid, 8% arginine, 8-9% alanine and 28% other amino acids.
      - Vitronectin (VTN or VN) is a glycoprotein of the hemopexin family which is abundantly found in serum, the extracellular matrix and bone
      - Laminins are high-molecular weight (~400 to ~900 kDa) glycoproteins of the extracellular matrix. They are a major component of the basal lamina (one of the layers of the basement membrane), a protein network foundation for most cells and organs. Laminins are heterotrimeric proteins that contain an α-chain, a β-chain, and a γ-chain (often found in five, four, and three genetic variants, respectively). The trimeric proteins intersect to form a cross-like structure that can bind to other cell membrane and extracellular matrix molecules:
         - The three shorter arms cand bind to other laminin molecules, forming larger structures (such as sheets).
         - The long arm binds to cells, anchoring the structure to the membrane.
      - Fibronectin is a high-molecular weight (~440 kDa) glycoprotein component of the extracellular matrix that binds to membrane-spanning receptor proteins called integrins, in addition to other extracellular matrix proteins such as collagen, fibrin, and heparan sulfateproteoglycans (e.g. syndecans). Two types of fibronectin are present in vertebrates: soluble plasma fibronectin (a major protein component of blood plasma) and insoluble cellular fibronectin (a major component of the extracellular matrix).
- Functional components, that can enable the ink-to form a stable 3-D structure or to enable cell-colonization:
   ∘ A crosslinker (binding/crosslinking/clotting protein):
      - Fibrinogen is a large, fibrous, non-globular and soluble glycoprotein that is involved in blood clotting, as thrombin converts it into insoluble fibrin molecule, in the presence of Ca²⁺ via intermolecular interactions. Thrombin converts fibrinogen to fibrin and then to a fibrin-based blood clot. Fibrin clots function primarily to occlude blood vessels to stop bleeding
         The fibrinogen protein is a 45 nm hexameric arrangement of peptide chains (alpha, beta, and gamma) connected by disulfide bonds, with a molecular weight of approximately 340 kDa. The fibrinogen protein consists of two outer D domains and a central E domain and is encoded by chromosome 4. Fibrin can form a fibrin scaffold as a network of protein that holds together and supports a variety of living tissues. It is produced naturally by the body after injury, but also can be engineered as a tissue substitute to speed healing. The scaffold consists of naturally occurring biomaterials composed of a cross-linked fibrin network and has a broad use in biomedical applications.
         Fibrinogen can further promote cell attachment and proliferation.

In some embodiments, the components can belong to more that one classification.
- Other:
   ∘ Crosslinking agent or factor, referring to a compound (or compounds) that induces bonding (or crosslinking) via at least one component of the ink. The ink may include more than one crosslinking agent, with each agent creating binds to the same or different components. The bond can be covalent on ionic.
      In some embodiments, the crosslinker is a coagulating agent:
      - Thrombine is a serine protease that converts soluble fibrinogen into insoluble strands of fibrin. Thrombine attacks the N-terminus of the Aα and Bβ chains in fibrinogen to form individual fibrin strands plus two small polypeptides, fibrinopeptides A and B, derived from these respective chains. The individual fibrin strands aggregate to form a three-dimensional gel-like structure by polymerizing and crosslinking with other fibrin stands. Fibrin regulates the aggregation by possessing three low affinity binding sites (two in fibrin's E domain; one in the D domain) for thrombin; this binding sequesters thrombin from attacking fibrinogen.
         The processes happen in the presence of 2⁺ ions (such as calcium).
      - Cerastocytin, a thrombin-like serine protease, such as found in snake venom.
      - Calcium (Ca²⁺), often added as a salt (such as Calcium chloride, CaCl₂) is necessary for blood clotting and is also referred to as co-factor IV. Calcium binds to Thrombin and fibrinogen. It has been proposed that Ca²⁺ forms a Ca²⁺-dependent dimer that catalyzes fibrinogen proteolysis by thrombin.
      - 2⁺ Ions, such as calcium, magnesium, manganese, strontium and copper, that can also reversibly crosslink polysaccharides, such as Sodium alginate, in a rapid manor (crosslinking of Sodium alginate by calcium can be completed in 2-5 minutes).

The bio-ink may also comprise additional minor components, such as:
∘ Solvents, such as water-based buffers (such as PBS and HEPES) and glycerol-water solutions. In many cases the buffer is effective at pH 6.8 to 8.2 (pKa 7.55).
∘ Cells: the cells colonize the printed 3-D structure. In some embodiments, the cells are harvested from the subject/patent before the printing. In some embodiments, the cells are harvested from a donor.
   In some embodiments, the cells are subjected to a procedure before being adding to the printed structure, such as incubation etc.
∘ Additional ingredients:
   - Salts:
      - Calcium (Ca²⁺), added as Calcium chloride (CaCl₂), is necessary for blood clotting, Calcium is also referred to as co-factor IV. Calcium binds to Thrombin and fibrinogen. It has been proposed that Ca²⁺ forms a Ca²⁺-dependent dimer that catalyzes fibrinogen proteolysis by thrombin.
      - Divalent metal ions such as Mg²⁺, Mn²⁺ Be²⁺, Al²⁺ and Sr²⁺, often added as Chloride salts, has been show to interact with Thrombin and fibrinogen to facilitate the crosslinking process (MgCl₂, MnCl₂ and SrCl₂).
   - Acids (such as Acetic Acid and Sulphoric acid) and bases (such as NaOH), to be used as co-solvents and for regulating pH of the solution. Most acids and bases are used in aqueous solutions. In many embodiments, the solutions are in concentrations of <0.5M.
   - Additional (minor) components, that can be osmolytes (such as D-mannitol) or stabilizers (such as TWEEN) can be added. Some of these components, such as D-mannitol, assist in maintaining the bioink's osmolarity, and therefore the for the compatibility of mammalian cells in the bioink,

A general method for preparation of the bio-ink of the present invention:
a. Preparing a solution of Sodium alginate and gelatin;
b. Heating solution (in a range of 50-80°C);
c. Preparing a solution of fibrinogen;
d. Preparing a solution of collagen;
e. Mixing fibrinogen and collagen solutions
f. Adding cells to fibrinogen-collagen solution
g. Mixing cell, fibrinogen and collagen solution with Sodium alginate and gelatin solution

The final bio-ink product comprises the ingredients in a ratio of:

**Table 1: composition of bio-ink**

| Ingredient | % of ink components | Content Range |
|---|---|---|
| Sodium alginate | 23 (20-30) | 20-25mg/ml |
| Gelatin | 60 (55-65) | 40 to 50 mg/ml |
| Fibrinogen | 10 7-12) | 40 to 60 mg/ml |
| Collagen | 2 (1-3) | 2 to 3 mg/ml |
| | | |

| Solvents | % of solvent | |
|---|---|---|
| PBS | 90 | 800-900ul/ml |
| Glycerol | 10 | 100 to 200ul/ml |
| Acetic Acid | 0.1 | 10 to 20ul/ml |

General method for printing a structure, using the bio-ink of the present invention:
a. Preparing the bio-ink (according to the method disclosed above)
b. Setting printing head and template temperature
c. Preparing the crosslinking solution
d. Printing, conducted in conditions suitable for cell viability (temperature range of 20 to 40°C and pressure of 10 to 20 kPa)
e. Adding crosslinker solution to printed structure
f. Washing structure in a cell medium, such as Minimal Essential Medium (MEM) or other (synthetic) cell culture medium.
g. Incubate structure in a CO₂ incubator with in MEM.

The final product could be considered as a Hydrogel, a class of crosslinked polymeric substances capable of absorbing and retaining large quantities of water.

The final printed product comprises the ingredients in a ratio of:

**Table 2: composition of printed structure**

| Ingredient | % of Ink ingredients | Content Range (per 0.1ml) |
|---|---|---|
| Sodium alginate | 23 (20-25%) | 2-2.5mg |
| Gelatin | 60 (55-65%) | 4 to 5 mg |
| Fibrinogen | 10 (8-12%) | 4 to 6 mg |
| Collagen | 2 (1-3%) | 0.2 to 0.3 mg |
| Cells | 5 (2-10%) | 100,000 to 500,000 |
| | | |

| Additional components | | |
|---|---|---|
| Solvents (PBS, glycerol) | | 60-100ul |
| Acids (Acetic Acid) | Trace amount (0.01%) | 3-5ul |
| Crosslinking agent | 10 | 50 ul |
| Salts (CaCl₂) | 0.1 | |

The MEM that can be used to maintain cells in tissue culture and comprises salts (such as calcium chloride, potassium chloride, magnesium sulfate, sodium chloride, sodium phosphate and sodium bicarbonate), glucose, amino acids, and vitamins (such as thiamine (vitamin B₁), riboflavin (vitamin B₂), nicotinamide (vitamin B₃), pantothenic acid (vitamin B₅), pyrodoxine (vitamin B₆), folic acid (vitamin B₉), choline, and myo-inositol (originally known as vitamin B₈). In some embodiments, the MEM is Dulbecco's modified Eagle's medium (DMEM).

A more detailed method for the production of an ink would be:
1) Sterilizing the Sodium alginate and gelatin, using UV exposure for 20 min
2) Wash the magnetic bead with 100% ethanol for 30min and autoclave the magnetic beads, Scintillation Vials, 3 mL cartridges with tip caps, Nozzles, Female/female Luer lock adapter.
3) Mix the 9ml PBS and 1ml glycerol and filter through 0.45uM
4) Dissolve Sodium alginate (20 mg) and gelatin (40mg) in 1ml PBS:glycerol (9:1), and pin sterile scintillation vial having magnetic bead.
5) Heat the solution at 70 °C for 40 min with constant stirring.
6) Transfer the solution/mixture/gel to cartridges.
7) Centrifuge the cartridge (4000rpm for 10min) to remove air bubbles. The gel can be stored at 4°C.
8) Dissolve the fibrinogen (50 mg/ml) in PBS by shaking for 30min at 30°C.
9) Dissolve the collagen (1 mg/ml) in a 0.1M acetic acid solution.
10) Neutralized the collagen solution using 0.1N NaOH 2.2mg/ml to a pH of 6-7.
11) Mix the fibrinogen and collagen in 1:1 ratio and filter through 0.45uM. The solution of 300 ul comprises 7.5 mg Fibrinogen and 0.3mg collagen.
12) Harvest the cells and generate the cell pellet having appropriate number of cells (approximately 2 million to 8 million cells).
13) Dissolve the cell pellet in 300 ul of fibrinogen and collagen mixture (prepared in step 10) and transferred to 3 ml syringe (approximately 2 million to 8 million cells).
14) 1ml Sodium alginate and gelatin solution (step 7) in a sterile cartridge (3ml) is mixed with 0.3 ml of the cells, fibrinogen and collagen solution.
15) Set the temperate in a temperature-controlled print head and print bed.
16) Prepare template. In some embodiments, the template is a cylinder 5X1mm.stl.
17) Printing: Bio printing procedure: temperature 20-25°C, Nozzle-25G, Speed:1mm/sec, pressure-10-20 kPa, Infill density-0%, 3D model- cylinder 5X1.
18) Preparing crosslinking solution: 990 ul CaCl₂and 10 ul thrombine are dissolved in PBS. The solution can be stored at ~-20°c
19) Add crosslink solution (step 17) to the printed structure (step 16) and incubate for 2-5 min.
20) The printed structure is washed twice with DMEM Complete media (2X 500 ul).
21) Add 500 ul DMEM Complete media and transferred to CO₂ incubator. In some embodiments, the incubation is for a period of approximately 14 days

### Examples

### Material and methods: Cell culture: GBM, HCT116, HaCaT

HCT116: HCT116 (ATCC CCL-247) colon cells were grown at 37°C in a humidified 5% CO₂-95% air atmosphere. Cells were maintained in McCoy's 5a Modified Medium having 10% fetal bovine serum and 1% Penstrep. Cells were grown in 250ml cell culture flask by giving 10 ml complete media twice a weekly. On reaching 80-90% confluence, cells are detached using 1.5 ml Trypsin EDTA solution (0.25% trypsin and 0.05% EDTA) in 5 min, added 10 ml McCoys complete media. For Bioprinting 8 X 10⁶cells were mixed with 300 ul of collagen: fibrinogen.

GBM: A172 (ATCC^{®} CRL-1620^{™}) glioblastoma cells were grown at 37°C in a humidified 5% CO2-95% air atmosphere in T-75 flasks. Cells were maintained in complete DMEM Medium (with 10% FBS, 5ml pen-strep, 5ml L-glutamine, 100ul plasmocin).

The GBM 3d structures printed into 24 wells plate in complete DMEM media at concentration of 8 million cells per mL. After 24 hours incubation, the medium replaced to serum-free DMEM/F-12 (500µL per well).

### Procedure for preparation of ECM (1.8% Sodium alginate+gelatin+collagen+fibrinogen [AGCF] BioGel)

1) Sterilization of Sodium alginate and gelatin using UV exposure for 20 min
2) Wash the magnetic bead with 100% ethanol for 30min and autoclave the magnetic beads, Scintillation Vials, 3 mL cartridges with tip caps, Nozzels, Female/female Luer lock adapter.
3) 1.8% Sodium alginate= Dissolve the 39.6 mg Sodium alginate and 90mg gelatin in 2ml solvent (mix 1.8 ml pbs with 0.2ml glycerol and filter through 0.45uM) in sterile scintillation vial having magnetic bead.
4) Heat the solution at 70 °C for 40 min with constant stirring.
5) Transfer the gel to 3 mL cartridges with end and tip caps.
6) Centrifuge the cartridge at 4000rpm for 10min to remove air bubbles and Store at 4°C.
7) Dissolve the 50 mg/ml fibrinogen in PBS in shaker for 30min at 30°C.
8) Dissolve the 2.2 mg/ml collagen in 0.1M acetic acid and neutralized the solution using 1N NaoH.
9) Mix the fibrinogen and collagen in 1:1 ratio and filter through 0.45uM
10) Preparation of crosslinking agent: mix 990 ul CaCl₂ and 10 ul thrombine and keep cooled (in ice).
11) Harvest the cells and generate the cell pellet having appropriate number of cells (as indicated in results).
12) Dissolve the cell pellet in 300 ul fibrinogen and collagen mixture (prepared in step 9) and transferred to 3 ml syringe.
13) Take 1ml of 1.8% Sodium alginate in sterile 3ml cartridges and mix thoroughly using 3ml syringe having cells+fibrinogen+collagen.
14) Set the print head and print bed to 20°C.

### Bio printing procedure: Nozzle-25G, Speed-1mm/sec, pressure-20 kPa, Infill density-0%, 3D model- cylinder 5X1

15) Add crosslink agent (step 10) and incubate for 5-10 min, wash the structure with DMEM Complete media (2timesX 500 ul)
16) Add 500 ul DMEM Complete media and transferred to CO₂ incubator
17) After 3 days, aspirated media and added serum free DMEM media, changed media for every 3 days

Confocal image was taken at day 1 and then the following days to determine cell proliferation and organization. Cells were stained with DAPI (Fluoroshield with DAPI-F6057) and cell nuclei were detected.

### DAPI staining:

1) Transfer carefully the 3D structure from 24 well plate to confocal plate
2) Wash the structure with 500 ul PBS for 2 times
3) Add Dapi 2- 3 drops to cover whole structure under dark
4) Incubate in CO₂ incubator for 45 min
5) To Confocal Microscope

### Digestion procedure:

1) Dissolve 1.47 gm of trisodium citrate dehydrate in 10ml 0.5M EDTA and filter through 0.22 uM (tube 1)
2) Dissolve 0.3 gm sodium chloride in 10ml water (tube 2) and transfer 3 ml from tube 1 to 7 ml water and filter through 0.22 uM (tube 3)
3) Digestion solution 0.05M sodium citrate+0.05M EDTA: Take 9ml solution from tube 3- and 1-ml solution from tube 1, mix thoroughly and store in ice basket
4) Transfer the 3D str. To 1.5 ml vial and wash with PBS (500 ul X 2 times)
5) Add 200 ul digestion solution (step 3) and mix thoroughly using tip (10 to 20 times) until the str. Disappear completely
6) Add 200 ul DMEM complete media and centrifuge at 1300 rpm / 3min
7) Wash the pellate with 500ul PBS -2 times
8) Add 500 ul DMEM complete media and transfer the cell to 24 well plate
9) Next Day Aspirate the media and add 500 ul media to the attached cells

### Results:

### 1. Glioblastoma (GBM)

### 1.1 Microscopic observation

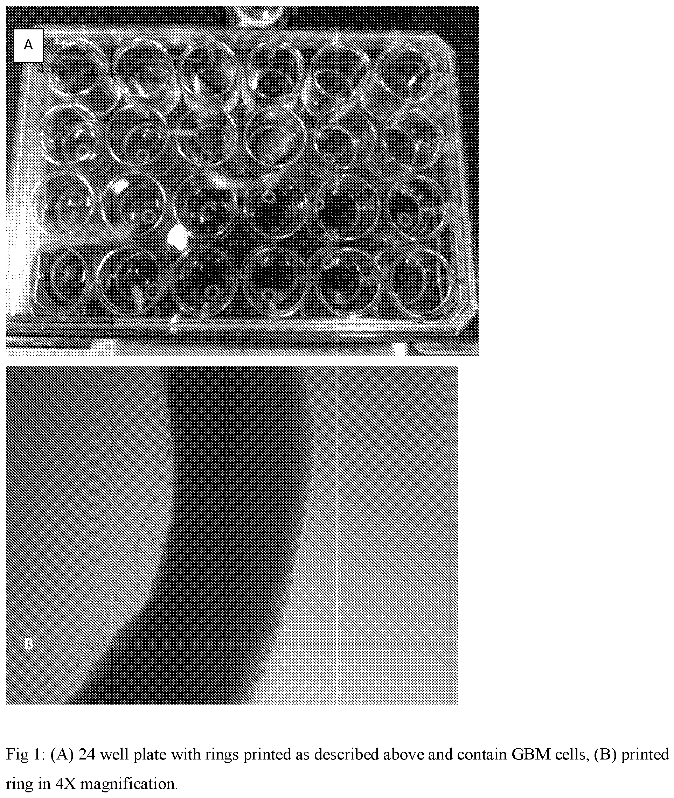

Reference is made to figure 1, demonstrating the pelleting and printing of 8 million cells in a circle structure (as described above).

The cells were stained with DAPI and observed, as optical sections, by confocal. Image acquisition was done Leica SP8 laser scanning microscope (Leica, Wetzlar, Germany), equipped with a solid-state laser with 405 nm light, HC PL APO CS 10x/0.40 objective (Leica, Wetzlar, Germany) and Leica Application Suite X software (LASX, Leica, Wetzlar, Germany). DAPI emission signals were detected with PMT detector in ranges of 415-490 nm ligh.

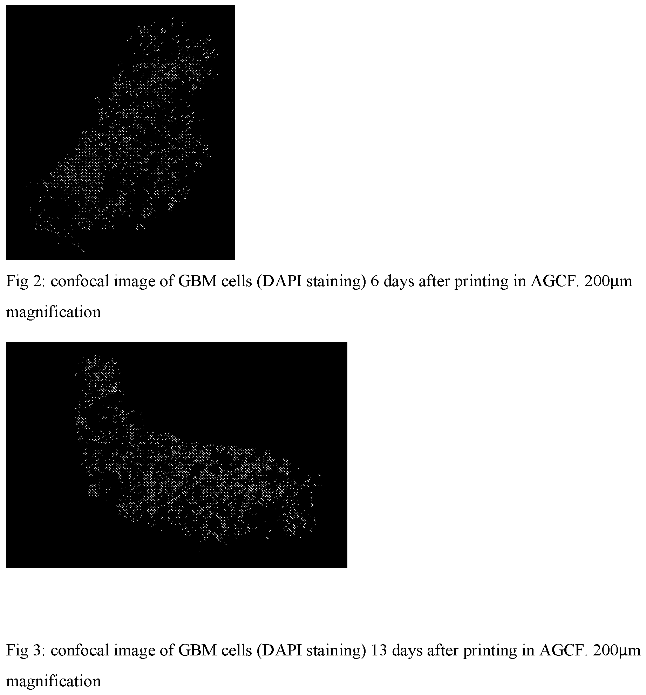

Reference is made to figure 2, demonstrating the homogenously spread of the cells through the printed structure at day 6.

Reference is made to figure 3, demonstrating the accumulation of cells at day 13, forming nets of accumulated cells.

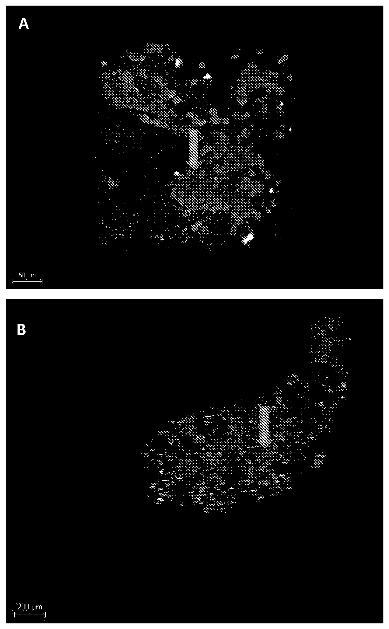

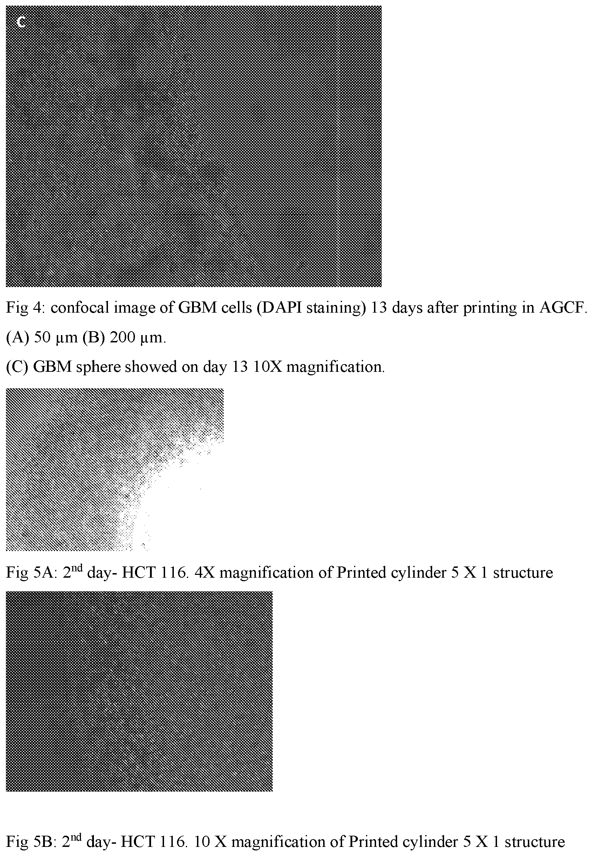

Reference is made to figure 4, demonstrating the presence of new secondary structures that contain relatively small and packed cells, formed in places of accumulated using a progenitor cell. The secondary structures (Fig 4, arrow) are usually referred to as colonies or spheres. These development processes are similar to those documented in 2D tissue cultures (Lopez-Bertoni H, Lal B, Li A, Caplan M, Guerrero-Cazares H, Eberhart CG, Quinones-Hinojosa A, Glas M, Scheffler B, Laterra J, Li Y. DNMT-dependent suppression of microRNA regulates the induction of GBM tumor-propagating phenotype by Oct4 and Sox2. Oncogene. 2015 Jul;34(30):3994-4004.) These spheres (neurospheres) are sub-populations of multipotent stem-like cells and efficiently propagate tumors in xenograft models, reflecting their self-renewing and tumor-propagating capacity. Evidences suggest that these stem-like cells have important role in maintaining tumor growth, therapeutic resistance and tumor recurrence (Lopez-Bertoni et al., 2015).

Our results suggest that GBM cells in 3D structures of AGCF develop similarly to GBM cells in 2D culture plates.

### 2. Colorectal cancer (CRC)

8 million cells were pelleted and printed in a circle structure as described above (Fig 1).

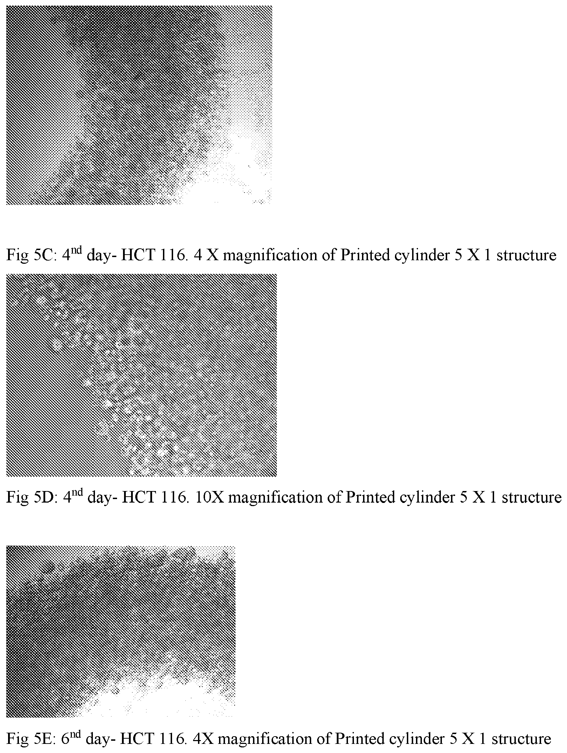

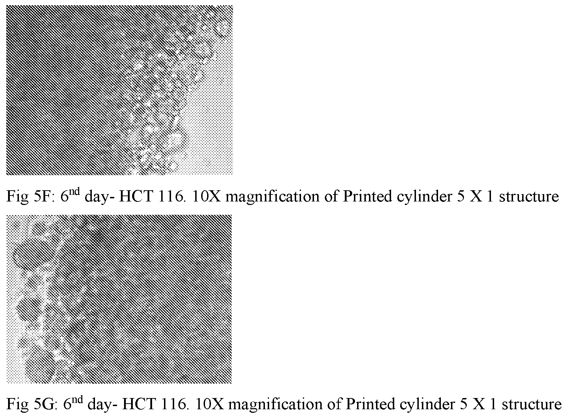

Bright field images of the printer structure are below (Fig 5).

## Claims

1. A bio-ink comprising:
a. a mixture of gelatin and sodium alginate;
b. at least one structural protein selected from a group consisting of collagen, vitronectin, laminins and fibronectin; and
c. fibrinogen;
wherein said mixture is pre-heated to a temperature in the range of 50-80°C.

2. The bio-ink of claim 1, wherein said at least structural protein is collagen, and the ratio of said gelatin to said collagen is approximately 15:1 to 25:1.

3. The bio-ink of claim 1, wherein the ratio of said fibrinogen to said collagen is at a ratio of 3:1 to 1:3.

4. The bio-ink of claim 1, additionally comprising cells.

5. The bio-ink of claim 4, wherein said cells are **characterized by** being viable/alive.

6. The bio-ink of claim 1, additionally comprising at least one crosslinking agent, wherein at least one of the following is true:
a. said crosslinking agent is selected from a group consisting of thrombin, calcium, copper, magnesium, manganese, strontium, barium, aluminum or a combination thereof; and
b. said crosslinking agent is a serine protease.

7. The bio-ink of claim 1, additionally comprising salts, acids, bases and buffer solutions, further wherein one of the following is true:
a. said salt is a calcium salt, a copper salt, magnesium salt, manganese salt, a strontium salt, a barium salt, an aluminum salt and a combination thereof; and
b. said salt is a chloride salt or a sulfate.

8. The bio-ink of claim 7, wherein said copper salt selected from a group consisting of copper (II) sulfate (CuSO4), copper bromide (CuBr₂), copper fluoride (CuF₂), copper carbonate (CuCO₃), Copper nitrate (CuNO₃)₂, Copper oxide (CuO), Copper acetate Cu(OAc)₂ and Copper azide Cu(N₃)₂.

9. The bio-ink of claim 1, **characterized by** at least one of the following:
a. viscosity in a range of 1-20000 Pa.S;
b. cell proliferation of ≥80%;
c. cell viability of ≥80%;
d. soluble in 0.05M EDTA+0.05M sodium citrate;
e. pH of 6.5-7.4; and
f. a boiling point of >100°C;

10. A method of producing a bio-ink, comprising steps of:
a. preparing a solution of sodium alginate and gelatin;
b. preparing a solution of fibrinogen;
c. preparing a solution of collagen;
d. mixing said fibrinogen and collagen solutions;
e. adding cells to said fibrinogen and collagen solution; and,
f. mixing said cell, fibrinogen and collagen solution with said Sodium alginate and gelatin solution;
wherein said step (a) comprises heating said solution of said sodium alginate and gelatin to a temperature in the range of 50-80°C.

11. The method of claim 10, additionally comprising at least one of the steps:
a. sterilizing said solutions;
b. filtering said solutions;
c. centrifuging said solutions; and
d. filtering said solutions.

12. A 3D structure, comprising:
a. a mixture of gelatin and sodium alginate;
b. at least one structural protein selected from a group consisting of collagen, vitronectin, laminins and fibronectin;
c. viable cells;
d. fibrinogen; and
e. at least one crosslinking agent,
wherein said structure supports cell development and formation of secondary structures, further wherein said mixture is pre-heated to a temperature in the range of 50-80°C.

13. The 3D structure of claim 12, **characterized by** at least one of the following:
a. viscosity in a range of 1-20000 Pa.S;
b. cell viability of ≥80%;
c. soluble in 0.05M EDTA+0.05M sodium citrate; and
d. stability at a temperature range of 35 to 42 °C;

14. A method of printing a structure, comprising steps of:
a. obtaining the bio-ink of claim 1;
b. obtaining a printer and a temperature-controlled printer head;
c. preparing said printer head;
d. printing said bio-ink;
e. adding at least one crosslinking agent; and
f. incubating said printed structure;
wherein said crosslinking agent coagulates said bio-ink;
further wherein said printing is **characterized by** at least one of the following:
a. a temperature in a range of 20-25°C;
b. a nozzle of 22G, 25G or 27G;
c. a speed of 1 to 3 mm/sec;
d. pressure of 10 to 20 kPa; and
e. an Infill density of <25%.

## Patentansprüche

1. Biotinte, die Folgendes:
a. eine Mischung aus Gelatine und Natriumalginat;
b. mindestens ein Strukturprotein, das aus einer Gruppe ausgewählt ist, die aus Kollagen, Vitronektin, Lamininen und Fibronektin besteht; und
c. Fibrinogen;
wobei die Mischung auf eine Temperatur in dem Bereich von 50-80 °C vorgewärmt wird.

2. Biotinte nach Anspruch 1, wobei das mindestens eine Strukturprotein Kollagen ist und das Verhältnis der Gelatine zu dem Kollagen ungefähr 15 : 1 bis 25 : 1 beträgt.

3. Biotinte nach Anspruch 1, wobei das Verhältnis des Fibrinogens zu dem Kollagen bei einem Verhältnis von 3 : 1 bis 1 : 3 vorliegt.

4. Biotinte nach Anspruch 1, die zusätzlich Zellen umfasst.

5. Biotinte nach Anspruch 4, wobei die Zellen **dadurch gekennzeichnet sind, dass** sie lebensfähig/lebend sind.

6. Biotinte nach Anspruch 1, die zusätzlich mindestens ein Vernetzungsmittel umfasst, wobei mindestens eines der Folgenden gilt:
a. das Vernetzungsmittel ist aus einer Gruppe ausgewählt, die aus Folgenden besteht: Thrombin, Calcium, Kupfer, Magnesium, Mangan, Strontium, Barium, Aluminium oder einer Kombination davon; und
b. das Vernetzungsmittel ist eine Serinprotease.

7. Biotinte nach Anspruch 1, die zusätzlich Salze, Säuren, Basen und Pufferlösungen umfasst, wobei weiter eines der Folgenden gilt:
a. das Salz ist Folgendes: ein Calciumsalz, ein Kupfersalz, Magnesiumsalz, Mangansalz, ein Strontiumsalz, ein Bariumsalz, ein Aluminiumsalz und eine Kombination davon; und
b. das Salz ist ein Chloridsalz oder ein Sulfat.

8. Biotinte nach Anspruch 7, wobei das Kupfersalz aus einer Gruppe ausgewählt ist, die aus Folgenden besteht: Kupfer(II)-sulfat (CuSO4), Kupferbromid (CuBr₂), Kupferfluorid (CuF₂), Kupfercarbonat (CuCO₃), Kupfernitrat (CuNO₃)₂, Kupferoxid (CuO), Kupferacetat Cu(OAc)₂ und Kupferazid Cu(N₃)₂.

9. Biotinte nach Anspruch 1, die durch mindestens eines der Folgenden gekennzeichnet ist:
a. Viskosität in einem Bereich von 1-20000 Pa·s;
b. Zellproliferation von ≥ 80 %;
c. Zelllebensfähigkeit von ≥ 80 %;
d. in 0,05 M EDTA + 0,05 M Natriumcitrat löslich;
e. pH von 6,5-7,4; und
f. einem Siedepunkt von > 100 °C.

10. Verfahren zur Herstellung einer Biotinte, das die folgenden Schritte umfasst:
a. Herstellen einer Lösung von Natriumalginat und Gelatine;
b. Herstellen einer Lösung von Fibrinogen;
c. Herstellen einer Lösung von Kollagen;
d. Mischen der Fibrinogen- und Kollagenlösungen;
e. Zugeben von Zellen zu der Fibrinogen- und Kollagenlösung; und
f. Mischen der Zell-, Fibrinogen- und Kollagenlösung mit der Natriumalginat- und Gelatinelösung;
wobei der Schritt (a) das Erwärmen der Lösung des Natriumalginats und der Gelatine auf eine Temperatur in dem Bereich von 50-80 °C umfasst.

11. Verfahren nach Anspruch 10, das zusätzlich mindestens einen der folgenden Schritte umfasst:
a. Sterilisieren der Lösungen;
b. Filtrieren der Lösungen;
c. Zentrifugieren der Lösungen; und
d. Filtrieren der Lösungen.

12. 3D-Struktur, die Folgendes umfasst:
a. eine Mischung aus Gelatine und Natriumalginat;
b. mindestens ein Strukturprotein, das aus einer Gruppe ausgewählt ist, die aus Kollagen, Vitronektin, Lamininen und Fibronektin besteht;
c. lebensfähige Zellen;
d. Fibrinogen; und
e. mindestens ein Vernetzungsmittel,
wobei die Struktur die Zellentwicklung und Bildung von sekundären Strukturen unterstützt, wobei weiter die Mischung auf eine Temperatur in dem Bereich von 50-80 °C vorgewärmt wird.

13. 3D-Struktur nach Anspruch 12, die durch mindestens eines der Folgenden gekennzeichnet ist:
a. Viskosität in einem Bereich von 1-20000 Pa·s;
b. Zelllebensfähigkeit von ≥ 80 %;
c. in 0,05 M EDTA + 0,05 M Natriumcitrat löslich; und
d. Stabilität bei einem Temperaturbereich von 35 bis 42 °C.

14. Verfahren zum Drucken einer Struktur, das die folgenden Schritte umfasst:
a. Erhalten der Biotinte nach Anspruch 1;
b. Erhalten eines Druckers und eines temperaturgesteuerten Druckkopfes;
c. Vorbereiten des Druckkopfes;
d. Drucken der Biotinte;
e. Zugeben von mindestens einem Vernetzungsmittel; und
f. Inkubieren der gedruckten Struktur;
wobei das Vernetzungsmittel die Biotinte koaguliert;
wobei weiter das Drucken durch mindestens eines der Folgenden gekennzeichnet ist:
a. eine Temperatur in einem Bereich von 20-25 °C
b. eine Düse von 22G, 25G oder 27G;
c. eine Geschwindigkeit von 1 bis 3 mm/Sek.
d. Druck von 10 bis 20 kPa; und
e. eine Fülldichte von > 25 %.

## Revendications

1. Encre biologique comprenant :
a. un mélange de gélatine et d'alginate de sodium ;
b. au moins une protéine structurelle choisie dans le groupe constitué par le collagène, la vitronectine, les laminines et la fibronectine ; et
c. du fibrinogène ;
dans laquelle ledit mélange est préchauffé jusqu'à une température dans la plage de 50-80°C.

2. Encre biologique selon la revendication 1, dans laquelle ladite au moins une protéine structurelle est le collagène, et le rapport entre ladite gélatine et ledit collagène va d'environ 15:1 à 25:1.

3. Encre biologique selon la revendication 1, dans laquelle le rapport entre ledit fibrinogène et ledit collagène se trouve selon un rapport allant de 3:1 à 1:3.

4. Encre biologique selon la revendication 1, comprenant en outre des cellules.

5. Encre biologique selon la revendication 4, dans laquelle lesdites cellules sont **caractérisées en ce qu'**elles sont viables/vivantes.

6. Encre biologique selon la revendication 1, comprenant en outre au moins un agent de réticulation, où au moins l'une des conditions suivantes est remplie :
a. ledit agent de réticulation est choisi dans le groupe constitué par la thrombine, le calcium, le cuivre, le magnésium, le manganèse, le strontium, le baryum, l'aluminium ou une combinaison de ceux-ci ; et
b. ledit agent de réticulation est une sérine protéase.

7. Encre biologique selon la revendication 1, comprenant en outre des sels, des acides, des bases et des solutions tampons, où l'une des conditions suivantes est également remplie :
a. ledit sel est un sel de calcium, un sel de cuivre, un sel de magnésium, un sel de manganèse, un sel de strontium, un sel de baryum, un sel d'aluminium et une combinaison de ceux-ci ; et
b. ledit sel est un sel chlorure ou un sulfate.

8. Encre biologique selon la revendication 7, dans laquelle ledit sel de cuivre est choisi dans le groupe constitué par le sulfate de cuivre (II) (CuSO₄), le bromure de cuivre (CuBr₂), le fluorure de cuivre (CuF₂), le carbonate de cuivre (CuCO₃), le nitrate de cuivre (CuNO₃)₂, l'oxyde de cuivre (CuO), l'acétate de cuivre Cu(OAc)₂ et l'azoture de cuivre Cu(N₃)₂.

9. Encre biologique selon la revendication 1, **caractérisée par** au moins l'une des propriétés suivantes :
a. viscosité dans la plage de 1-20 000 Pa.s ;
b. prolifération cellulaire ≥ 80% ;
c. viabilité cellulaire ≥ 80% ;
d. solubilité dans de l'EDTA à 0,05 M + citrate de sodium à 0,05 M ;
e. pH de 6,5-7,4 ; et
f. point d'ébullition > 100°C.

10. Méthode de production d'une encre biologique, comprenant les étapes :
a. de préparation d'une solution d'alginate de sodium et de gélatine ;
b. de préparation d'une solution de fibrinogène ;
c. de préparation d'une solution de collagène ;
d. de mélange desdites solutions de fibrinogène et de collagène ;
e. d'addition de cellules à ladite solution de fibrinogène et de collagène ; et
f. de mélange de ladite solution de cellules, de fibrinogène et de collagène avec ladite solution d'alginate de sodium et de gélatine ;
dans laquelle ladite étape (a) comprend le chauffage de ladite solution dudit alginate de sodium et de ladite gélatine jusqu'à une température dans la plage de 50-80°C.

11. Méthode selon la revendication 10, comprenant en outre au moins l'une des étapes :
a. de stérilisation desdites solutions ;
b. de filtration desdites solutions ;
c. de centrifugation desdites solutions ; et
d. de filtration desdites solutions.

12. Structure 3D, comprenant :
a. un mélange de gélatine et d'alginate de sodium ;
b. au moins une protéine structurelle choisie dans le groupe constitué par le collagène, la vitronectine, les laminines et la fibronectine ;
c. des cellules viables ;
d. du fibrinogène ; et
e. au moins un agent de réticulation ;
ladite structure favorisant le développement cellulaire et la formation de structures secondaires, où, en outre, ledit mélange est préchauffé jusqu'à une température dans la plage de 50-80°C.

13. Structure 3D selon la revendication 12, **caractérisée par** au moins l'une des propriétés suivantes :
a. viscosité dans la plage de 1-20 000 Pa.s ;
b. viabilité cellulaire ≥ 80% ;
c. solubilité dans de l'EDTA à 0,05 M + citrate de sodium à 0,05 M ;
d. stabilité dans une plage de température comprise allant de 35 à 42°C.

14. Méthode d'impression d'une structure, comprenant les étapes :
a. d'obtention de l'encre biologique selon la revendication 1 ;
b. d'obtention d'une imprimante et d'une tête d'impression à température contrôlée ;
c. de préparation de ladite tête d'impression ;
d. d'impression de ladite encre biologique ;
e. d'addition d'au moins un agent de réticulation ; et
f. d'incubation de ladite structure imprimée ;
où ledit agent de réticulation coagule ladite encre biologique ;
ladite impression étant en outre **caractérisée par** au moins l'une des propriétés suivantes :
a. température dans la plage de 20-25°C ;
b. buse de 22G, 25G ou 27G ;
c. vitesse allant de 1 à 3 mm/sec ;
d. pression allant de 10 à 20 kPa ; et
e. densité de remplissage < 25%.
